# EUROPEAN PATENT SPECIFICATION

(11) **EP 4 111 199 B1**
(45) Date of publication and mention of the grant of the patent: **25.06.2025**
(21) Application number: 21714665.3
(22) Date of filing: 27.02.2021
(51) Int. Cl.: G01N 33/543, G01N 33/68

(54) **COLORIMETRIC DETECTION SYSTEM FOR RAPID DETECTION OF INFECTIOUS PULMONARY DISEASES AND A FACE MASK WITH SAID COLORIMETRIC DETECTION SYSTEM**
KOLORIMETRISCHES DETEKTIONSSYSTEM ZUR SCHNELLEN DETEKTION INFEKTIÖSER LUNGENERKRANKUNGEN UND GESICHTSMASKE MIT DIESEM KOLORIMETRISCHEN DETEKTIONSSYSTEM
SYSTÈME DE DÉTECTION COLORIMÉTRIQUE PERMETTANT LA DÉTECTION RAPIDE DE MALADIES PULMONAIRES INFECTIEUSES ET MASQUE FACIAL DOTÉ DUDIT SYSTÈME DE DÉTECTION COLORIMÉTRIQUE

(30) Priority: 29.02.2020 US 202062983620 P
(43) Date of publication of application: 04.01.2023
(73) Proprietor: Alekhmimi, Nuha Khalid, Riyadh, 12331 4624 (SA)
(72) Inventor: Alekhmimi, Nuha Khalid, Riyadh, 12331 4624 (SA)
(74) Representative: Hepp Wenger Ryffel AG
(86) International application number: PCT/IB2021/051658
(87) International publication number: WO 2021/171268

(56) References cited:
- WO-A1-2009/126336
- WO-A2-98/29727
- KR-A- 20170 063 000
- US-A1- 2010 092 944
- HIDEKAZU NISHINO ET AL: "Selective Protein Capture by Epitope Imprinting", ANGEWANDTE CHEMIE INTERNATIONAL EDITION, vol. 45, no. 15, 3 April 2006 (2006-04-03), pages 2392 - 2396, XP055009776, ISSN: 1433-7851, DOI: 10.1002/anie.200503760
- GUERREIRO ANTÓNIO ET AL: "Influence of Surface-Imprinted Nanoparticles on Trypsin Activity", ADVANCED HEALTHCARE MATERIALS, vol. 3, no. 9, 20 March 2014 (2014-03-20), DE, pages 1426 - 1429, XP055799200, ISSN: 2192-2640, DOI: 10.1002/adhm.201300634
- CHUA MING HUI ET AL: "Face Masks in the New COVID-19 Normal: Materials, Testing, and Perspectives", RESEARCH, vol. 2020, 7 August 2020 (2020-08-07), pages 1 - 40, XP055787873, Retrieved from the Internet <URL:http://downloads.spj.sciencemag.org/research/2020/7286735.xml> DOI: 10.34133/2020/7286735

## Description

The present invention relates to a colorimetric detection system for rapid detection of lung diseases, a colorimetric mask comprising said detection system, a method for producing a colorimetric detection system, a method for producing a mask comprising said detections system and the use of dyed microsphere for a colorimetric detection system.

Pulmonary infections are global health problems that can be spread anywhere. Spreading is in particular a problem in regions where many people live in cramped conditions. Likewise, seasonal festivities, such as carnival, or religious events, such as pilgrimages, pose a challenge in preventing the spread of diseases. Viral and bacterial microbes play a major role in lung infections and different attempts have been undertaken to prevent spreading of these microbes. One way to prevent spreading would be a rapid detection method and isolation of infected persons. Another relatively efficient way of preventing spreading is the use of facial mask that cover mouth and nose. If a patient with a lung disease is coughing or sneezing into the mask, viruses and microbes such as bacteria are caught in the layers of the mask preventing their uptake by another person standing close to the patient.

In addition to preventing the transmission of pathogens, it would also be desirable to be able to detect the symptom-causing disease as quickly and efficiently as possible. Indicator systems are known that are based on color changes. For example, Schaude et al (Sensors 2017, 17, p. 1365) describes indicator cotton swabs for a faster and less expensive way to obtain information about a wound status. The method is based on cotton swabs covalently functionalized with an indicator dye that changes color upon pH change visible to the naked eye. The pH can be used to determine the efficiency of wound healing. In case the pH is not optimal for wound healing, a distinct treatment to change the wound pH can be applied.

However, this method is only limited to pH changes and difficult to apply to detection systems for determining an infection based on pathogens. Further, in some cases color changes has to be recorded using digital photography which is time consuming.

US 2019/0309357 A1 describes a detection system comprising a CRISPR system comprising an effector protein and one or more guide RNAs designed to bind to corresponding target molecules; an RNA masking construct and one or more detection aptamers coupled with a detectable ligand. Enzymes generate a detectable signal by releasing the detectable ligand form the molecule which can be detected by e.g. fluorescence. The detection system can be prepared in freeze-dried format for convenient distribution and point-of-care. A comparable system has been applied to face mask (according to www.businessinsider.com). However, the system still needs hours to obtain a diagnostic signal. Further, the detection system is based on PCR techniques, which is rather expensive. Fluorescence measurements are susceptible to interference leading to noise signals thus complicating the analysis.

US 2010/092944 describes a colorimetric system for the detection of lung disease comprising antibodies coupled to a substrate for capturing a lung disease specific antigen characterized in that the colorimetric system further comprises dyed microspheres modified with the antibodies for visually detecting lung disease specific antigen wherein the antibodies are specific to an antigen of influenza virus and the solid support substrate is a hydrophilic material or cellulose.

Wo 2009/126336 describes a similar system with the same components, an antibody coupled to a substrate for capturing a lung disease antigen and further comprising a dyed microsphere modified with antibodies.

It is thus at least one objective of the present invention to overcome the drawbacks of the prior art. It is in particular an objective of the present invention to provide a colorimetric detection system for rapid detection of lung diseases. It is another objective of the present invention to provide a face mask with said colorimetric detection system that allows a rapid and reliable diagnosis of lung disease while, at the same time, preventing the release of lung disease-causing pathogens into the environment.

The objectives are solved by the subject-matters of the independent claims. Specific embodiments of the invention are described in the dependent claims.

A first aspect of the invention relates to a colorimetric detection system for rapid detection of lung diseases. The detection system comprises antibodies and/or aptamers coupled to a substrate for capturing a lung disease specific antigen. The colorimetric system further comprises dyed microspheres modified with the antibodies and/or aptamers for visually detecting the lung disease specific antigen. The colorimetric detection system is further characterized in that the substrate comprises a protease, preferably a matrix-metalloproteinase.

Antibodies are large Y-shaped proteins used by the immune system to identify pathogenic bacteria or viruses. The antibody recognizes a unique molecule of the pathogen, called the antigen. Aptamers are oligonucleotide or peptide molecules that bind to a specific target molecule. They can replace antibodies or used along antibodies for the same target.

The antibodies and/or aptamers coupled to the substrate are preferably coupled by covalent linkage and can be considered as capture antibodies and/or aptamers.

The modified dyed microspheres are preferably attached to the substrate by physical adsorption, thus being less strongly bound to the substrate than the capture antibodies/aptamers. However, they may also be bound by covalent linkage to the substrate. The antibodies and/or aptamers attached to the dyed microsphere are preferably of the same kind as the capture antibodies/aptamer but show at least the same antigen specificity as the capture antibodies/aptamers. During the description of the present invention the antibodies attached to the dyed microsphere may be referred to as detection antibodies and/or detection aptamers.

With "dyed" every way of obtaining a colored microsphere is meant. For example "dyed" can refer to attached color-causing ligands; color pigments, compounds or dyes enclosed in the microsphere or with dye coated microspheres. Suitable dyes are for example Polybead^{®} dyed microsphere obtainable by Polysciences Inc. The Polybeads may have different colors such as blue, green, yellow, red, pink, orange. The Polybead^{®} microsphere can be Polybead^{®} Polystyrene Microsphere, in the description also referred to as polystyrene microsphere or microsphere. The dyed microspheres can be provided as a particle suspension with a concentration of 2.5 % solid per 100 mL. The suspension may comprise particle diameters in the range of 0.05 µm to 90 µm. Particles with a diameter in the range of 0.5 to 90 µm account for 10 % or less of the solids in the suspension. The microspheres can be functionalized with carboxyl-, hydroxyl-, or amine groups for coupling of the antibodies and aptamers.

Preferably, the dyed microspheres used for coupling with the antibodies and/or aptamers have a mean particle diameter of less than 6 µm, preferably even less than 3 µm, even more preferably less than 1 µm and most preferably less than 500 nm. The individual microspheres in the colorimetric detection system can have different nominal particle diameter, but preferably do not exceed 6 µm. The sphericity of the dyed microspheres is preferably > 0.9, more preferably > 0.93, most preferably > 0.96. Individual microspheres with a diameter of less than 6 µm are not visible to the naked human eye and only allow detection upon agglutination thus providing a simple and effective detections system.

The substrate further comprises a protease, preferably a matrix-metalloproteinase. Preferably, the protease is coupled to the substrate by functional groups such as carboxyl-, hydroxyl- or amine groups present on the substrate. Other proteases are also possible, for example PR3 or Elastase.

The protease has the advantage that a pathogen specific antigen coupled to the respective antibodies or aptamer are cleaved easier into smaller fragments (peptides and amino acids) thus allowing more detection antibodies and/or aptamers to agglutinate to a larger network and thus allowing a faster detection.

The substrate can comprise discrete sections, each section being modified with antibodies and/or aptamers different from one of the other section for capturing a lung disease specific antigen different from the one captured in one of the other section. Thus, a detection system is provided that allows the detection of different pathogens simultaneously. Preferred are two to four discrete sections, even more three sections. However, the detection system can comprise even more discrete sections, being only limited by the size of the substrate to allow visual detection.

For example, one discrete section may comprise antibodies and/or aptamers specific for a pneumonia inducing bacteria while the other discrete section comprises antibodies and/or aptamers specific for a flue inducing antigen. The dyed microsphere may have different colors in order to distinguish the different diseases.

The antibodies and/or aptamers can be influenza virus antibodies and/or aptamers; or a pneumonia or pneumonia-inducing antibodies and/or aptamers.

In general, antigens that may be detectable by the colorimetric detection system are for example related to: viruses causing COVID-19, MERS, SARS, Flue A, Para Flue A Flue B, Para Flu B; bacteria such as Streptococcus, pneumonia, legionella, tuberculosis; or fungus such as actinomyces, aspergillus, candida spp. The list is not to be understood as exhaustive as basically every disease can be detected of which specific antibodies/aptamers are available.

Preferably, the substrate is a solid support. The substrate can be a hydrophilic material and/or oxidized cellulose. The substrate can be a cellulose based, paper-based or synthetic material. For example, tissue paper, cotton or functionalized plastic material can serve as substrate. The substrate can basically have any shape. Due to its easy handling the strip format is preferred. The substrate may have functional groups to allow coupling of the capture antibodies and/or aptamers, for example carboxyl-, hydroxyl- or amine groups, preferably by conventional carbodiimde coupling techniques. The carbodiimide technique is in particular used to couple proteins and peptides via their n-terminus to carboxylic groups to form stable conjugates. The hydrophilicity allows the uptake of body liquids in the substrate. Basically, any hydrophilic substrate that provides the above-mentioned conditions can be used. Further examples are silica or metal hydroxide such as aluminium hydroxide.

Without being bound to theory, the working principle of the colorimetric system can be explained as follows: If a sample of salvia, sputum or nasal muscus of an infected person is applied to the substrate the substrate is humidified. The pathogen specific antigen is caught by the capture antibodies or aptamers covalently attached to the substrate. The protease allows cleavage of the antigen into smaller fragments for an efficient agglutination of detection antibodies. The humidified substrate allows movement of the detection antibodies. The detection antibodies or aptamers, preferably attached to the substrate by physical adsorption, will attach to the pathogen/antivirus and may form agglutinated network. The agglutination of the detection antibodies/aptamers causes an agglutination of the dyed microsphere resulting in the color appearance of the respective dyed microspheres to the naked eye and thus allowing visual detection of the disease. Single dyed microsphere may be undetectable by the naked eye as described further above.

The colorimetric detection system provides a simple, efficient and fast way of detecting lung infections. The system can be easily prepared with low costs.

Preferably, the color appears within the first 30 min after applying the sample to the substrate, preferably between 1 and 10 min after application and even more preferably between 1 to 5 min after application and most preferably between 1 and 3 min after application.

Thus, the detection system does response faster than other known method such as fluorescent measurements and is less prone to errors. Further, no additional equipment is used for detection allowing its application everywhere.

A second aspect of the invention relates to a colorimetric face mask for rapid detection of lung diseases. The face mask is preferably designed to cover the mouth and the nose of a person.

The face mask comprises a first layer comprising the colorimetric detection system as previously described.

Advantageously, the first layer can be made from the same material as the substrate. The substrate itself can serve as the first layer. The first layer can also be made of material different from the substrate. For example, the first layer can comprise a hydrophobic material that prevents the adsorption of aqueous body liquids such as salvia. The first layer can be polypropylene or a hydrophobised material. The first layer can be impregnated material. The substrate can be attached to the first layer, for example by sewing, ticking or gluing.

The face mask can comprise a second outer layer for protection against external pathogenic factors, preferably comprising a synthetic polymer such as polypropylene. The outer layer can be obtained by polymeric fibres for exampling by spinning or weaving. Preferably, the outer layer is made of a hydrophobic material to avoid the uptake of pathogen-containing fluids such as aerosols, salvia or mucus of persons standing nearby. The outer layer can comprise impregnated material. Most preferably, the outer layer is impermeable to microparticle but allows easy breathing of the user.

The impregnated material can be any textile or paper material, of natural or synthetic origin, that has been treated with resins or other substances known in the art, or even has been guanidine-functionalized, in order to avoid water uptake and/or to obtain an antimicrobial effect.

The outer layer protects the user from being infected with a lung disease by others. At the same time is also prevents a false detection due to pathogens present in the environment instead of being present in the patient's body liquid. Further, it also avoids the visual detection by others which may lead to panic or the like.

With "pathogenic factors" all disease-causing pathogens, microbes and small particles are meant, in particular viruses, bacteria, fungus, dust and smog.

The working principle of the face mask can be explained similar as done with regard to the colorimetric detection system with the difference that the pathogen specific antigen containing sample is provided by sneezing or coughing into the mask. The coughing or snoozing brings the salvia, sputum or nasal muscus to the mask and humidifies the substrate. The humidified substrate allows movement of detection antibodies to the antigen and the agglutination to the network allowing. The agglutination of the detection antibodies/aptamers causes an agglutination of the dyed microsphere resulting in the color appearance of the respective dyed microspheres to the naked eye and thus allowing visual detection of the disease. Single dyed microsphere may be undetectable by the naked eye as described further above.

The colorimetric mask offers a cheap, fast and reliable method of detection a patient's or any other infected person's lung disease and at the same time prevents the spreading of the pathogens, thus lessen the risk of disease spreading.

The color can appear within the first 30 min after release of the pathogen by e.g. sneezing or coughing, preferably between 1 and 10 min after release and even more preferably between 1 to 5 min after release and most preferably between 1 and 3 min.

Preferably, the first layer comprises the colorimetric detection system with two or more discrete sections for the detection of different pathogens as previously described. For example, one discrete section may comprise antibodies and/or aptamers specific for a pneumonia inducing bacteria while the other discrete section comprises antibodies and/or aptamers specific for a flue inducing antigen. The dyed microsphere may have different colors in order to distinguish the different diseases.

Such a mask allows the detection of several lung diseases and thus no separate mask for each kind of infection has to be provided.

The colorimetric detection system can be located in the centre of the first layer, preferably in a circular arrangement. With "centre" is meant the middle of a plane forming cloth. Thus, the colorimetric detection system is closest to the mouth and nose, where the highest concentration on pathogen can be expected and thus leading to a fast detection. If more than one discrete section is used, the detection system is preferably arranged in a circle and divided into pie-shaped sections. However, other shapes may also be possible as long as a contact between pathogen and detection system can be ensured.

The colorimetric mask can comprise a third inner layer for hiding the result of the visually detected lung disease. The third inner layer is preferably permeable to fluids and preferably comprises a cotton based material. Permeability can thus either be achieved by the material itself or by providing fluid passage, such as holes, in the third layer to allow contact of the pathogen containing body fluid with the detection system in the first layer. In principle, all material mentioned with regard to the first and second layer can be used for the third layer.

The third layer avoids the visual detection of the disease by the patient himself. Most preferred, the visual appearance of the colors can only be interpreted by medical staff and thus avoiding revelation to the patient or surrounding people which may cause panic or fear.

The mask may comprise fixation loops for fixing the mask to the head. The loops can be handcrafted rubber strings with two metal copper rings inside, rubber bands or cords for closing at the back of the head. This way, the mask is holed in place and wearing is comfortable. The mask can thus be worn for a couple of hours.

A third aspect of the invention refers to a method for producing a colorimetric system as previously described. The method comprises the steps of
- Providing a substrate with reactive sites towards antibodies and/or aptamer,
- coupling of antibodies and/or aptamers specific for a lung disease causing antigen to the reactive sites,
- blocking of non-reacted active sites,
- attaching dyed microspheres modified with the antibodies and/or aptamers to the substrate, by physical adsorption, preferably by immersing the substrate into a solution comprising the modified dyed microspheres, characterized in that a protease, preferably a matrix metalloproteinase, is coupled to the substrate.

The substrate may be a hydrophilic material already containing the reactive sites or the substrate may have been modified in a previous step. The substrate can originate from cellulose which has been oxidized. The substrate can be oxidized cellulose. The substrate can be any other hydrophilic material that provides reactive sites towards antibodies and/or aptamers.

The antibodies and/or aptamers may be coupled to the substrate via the reactive groups. The reactive groups can be carboxyl-, hydroxyl-, amine- or sulfuric groups. Carboxyl groups are preferred. Coupling of the antibodies and aptamers can be conducted by carbodiimide crosslinking chemistry known in the art. In general, any coupling method that allows the N- or C-terminus of the antibody/aptamer to be coupled to the substrate is possible.

Blocking of non-reactive sites is preferably obtained by using bovine serum albumin (BSA).

Attachment of the dyed microspheres modified with the antibodies and/or aptamers to the substrate is preferably obtained by physical adsorption. Most preferably, the substrate can be immersed into a solution comprising the modified dyed microspheres. Details to the microsphere have been previously provided and apply here analogously. After immersing the substrate into the solution, the substrate is stained in the color of the dyed microsphere. Washing the substrate with buffer solution will remove excess dyed microsphere resulting in an essentially colorless substrate. Even though not visible to the naked eye, microspheres will still be attached the substrate by preferably physical adsorption.

Other methods then immersing are also possible. For example, the solution with the dyed microsphere modified with the antibodies and/or aptamers can be applied locally to the substrate, e.g. by drip on, spray on or the like. This is in particular advantageous if more than one antibody/aptamer system is applied to different discrete sections in the substrate.

The dyed microsphere modified with antibodies and/or aptamers are preferably obtained by a separate step. Conventional dyed polystyrene microspheres can be obtained from Polyscience Inc. (USA). Non-modified dyed microspheres can have a mean diameter of less than 6 µm, preferably less than 3 µm, even more preferably less than 1 µm and most preferably less than 500 nm. The sphericity of the non-modified dyed microspheres is preferably > 0.9, more preferably > 0.93, most preferably > 0.96.

The microspheres can be functionalized with carboxyl groups, but also hydroxyl- or amine groups are possible. Attachment of antibodies and aptamers can be conducted by using carbodiimide coupling techniques known in the art and previously described. After attaching the modified dyed microspheres to the substrate, the substrate can be dried before use.

The capture antibody is coupled to the substrate together with a protease, preferably a matrix metalloprotease. But also other proteases fulfilling the respective function are suitable. The coupling can be obtained simultaneously with the antibodies/aptamers, preferably by using horseradish peroxidase (HRP), or in sequence.

The method can provide the step of coupling two or more different antibodies and/or aptamers each specific for another lung disease causing antigen to the substrate. Antibodies and/or aptamers for one specific antigen are preferably coupled in a discrete section of the substrate. Coupling of capture antibodies and/or aptamers will preferably occur through chemical reaction such as carbodiimide crosslinking as previously described.

A fourth aspect of the invention relates to a method for producing a colorimetric mask as previously described. The method comprises the step of providing a first layer with a colorimetric detection system obtainable by a method as previously described.

The first layer can be made from the same material as the substrate. The substrate can even be the first layer itself. The first layer can also be made of material different from the substrate. For example, the first layer can comprise a hydrophobic material that prevents the adsorption of aqueous body liquids such as salvia, sputum or nasal muscus. The first layer can be polypropylene, hydrophobised material or any previously described material. The substrate can be attached to the first layer, for example by sewing, ticking or gluing.

The colorimetric detection system can be arranged in the centres of the first layer, meaning in the centre of the cloth forming the plane. Discrete sections of the colorimetric detections system can be arranged cicular and in pie-shape. But also other locations and shapes are possible as long as a contact between capture antibody/aptamer and antigen is provided.

Another aspect of the invention refers to the use of dyed microsphere modified with antibody and/or aptamer in a colorimetric detection system as previously described.

The invention will be described in more details with regard to the following figures and examples. The invention is not limited to these specific embodiments.

It shows:
- Figure 1:: A detection system according to the invention.
- Figure 2A:: A face mask according to the invention from the front side.
- Figure 2B:: The mask of figure 2A from the opposite side in a perspective view.
- Figure 3:: Preparation method of the colorimetric detection system.
- Figure 4:: Working principle of the colorimetric detection system.

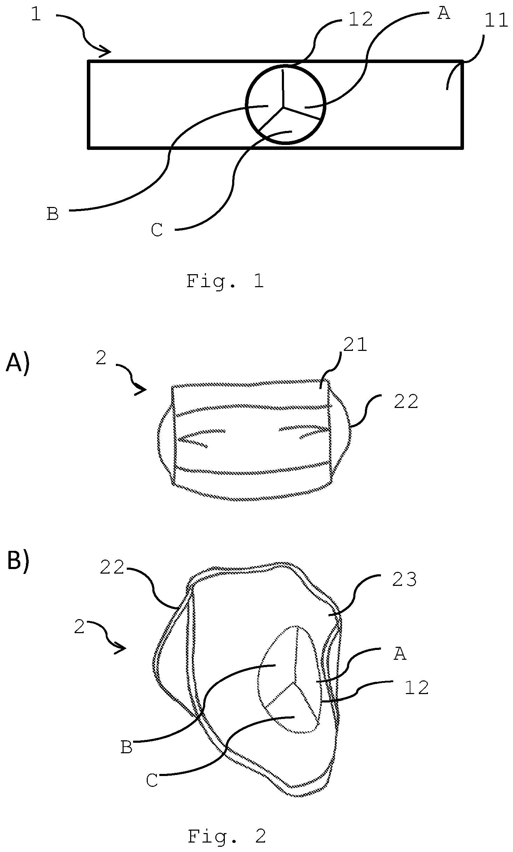

Figure 1 shows a colorimetric detection system 1 according to the invention. The colorimetric detection system 1 comprises a substrate 11, here in form of a strip. In the centre of the strip 11 is the detection area 12 in form of a circle with discrete detection sections A, B, C. Each detection section A, B and C are able to detect another pathogen specific antigen. For example, section A can comprise influenza virus antibodies/aptamers coupled to blued-dyed microsphere. Section B can comprise pneumonia antibodies/aptamer coupled to green dyed microsphere. Section C can comprise Covid-19 antibodies/aptamer coupled to red dyed microsphere.

If a patient's sample is applied to the detection system 1 and the patient is infected with influenza virus, the detection section A will change from colorless to blue while the other sections B and C remain colorless.

If a patient's sample is applied to the detection system 1 and the patient is infected with pneumonia, the detection section B will change from colorless to green while the other sections A and C remain colorless.

If a patient's sample is applied to the detection system 1 and the patient is infected with Covid 19, the detection section C will change from colorless to red while the other sections A and B remain colorless.

Figure 2A shows the face mask 2 according to the invention from the front with the outer layer 21 and the fixation loops 22 on each side for fixation on the ears.

Figure 2B shows the mask 2 from the backside, meaning the side facing mouth and nose of the user. The figure shows the first layer 23 comprising the detection area 12. Thus, in this specific example, the first layer also forms the substrate of the colorimetric detection system. The detection area 12 is provided as a circle in the centre of the mask 2, being the region closest to mouth and nose when in use. The detection area 12 is divided into three different pie-shaped sections A, B, C. Each section will be coupled with another antibodies/aptamer system specific for one particular antigen thus providing the possibility of detecting three different pathogen specific antigen with one mask.

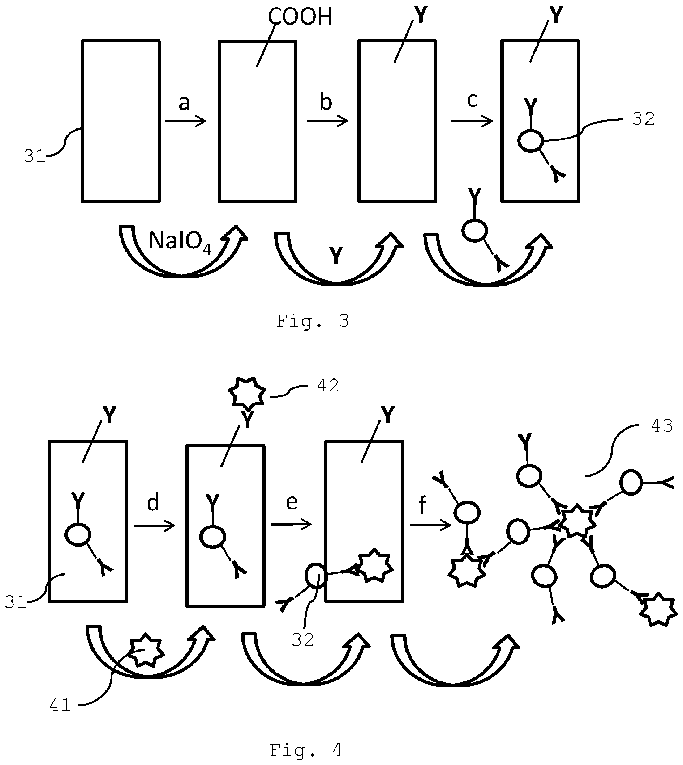

Figure 3 shows the modification of the substrate. Firstly, a cellulose substrate 31 is provided which is oxidized with sodium periodate (step a). Secondly, capture antibodies or aptamers or both are added to the oxidized cellulose and coupled by known carbodiimide coupling techniques to the cellulose (step b). Step b also comprises the step of coupling protease to the cellulose using horseradish peroxidase (not shown). Previously prepared dyed microsphere modified with the antibodies/aptamers (detection antibodies) 32 are attached to the substrate by immersing the cellulose substrate into a solution containing the dyed microspheres (step c). The dyed microsphere are preferably obtained by reaction of colored polystyrene microspheres with free carboxyl groups with the respective antibodies and/or aptamers by the previously mentioned carbodiimide coupling techniques known in the art.

Then, the substrate 31 is dried, attached to a first layer of a mask or being the first layer itself and mounted together with an outer layer 21 to the colorimetric mask 2 of figure 2.

Figure 4 shows the working principle of the colorimetric detection system. When salvia or nasal muscus sample is applied to the substrate, the pathogen specific antigen 41 will be captured by the capture antibody/aptamer 42 (step d). The protease cleaves the antigen 41 into smaller fragments (step e), thus allowing more detection antibodies 32 to be agglutinated around the antigen 41 leading to a agglutinated network 43. The network 43 comprises sufficient detection antibodies 32 to become visible to the naked eye. Movement of the detection antibodies 32 to the antigen's 41 reactive sites is preferably achieved through capillary effects upon humidification of the substrate.

### EXAMPLE

### 1. Reagents

Matrix metalloproteinase-2 (MMP2) was synthesized by Pepmic Co. (Suzhou, Jiangsu, China).

Sodium phosphate and monopotassium phosphate, Tris-Base, Sodium acetate; bovine serum albumin (BSA), sodium chloride, phosphate buffered saline (PBS), ethyl-3-(3-dimethylaminopropyl)-carbodiimide (EDC) , N-hydroxysuccinimide (NHS), ethylenediaminetetraacetic acid (EDTA) phosphate buffered were all purchased from Sigma-Aldrich (Darmstadt, Germany). Carboxylic acid functionalized microspheres of different colors (orange and blue) were obtained from Polysciences Inc. (Polysciences Inc., Warrington, PA).

All Aptameres of pathogen were provided from Aptamer Sciences (Aptamer Sciences Inc, Korea).

All antibody and antigen for testing the system were provided from abcam (abcam, UK), Sodium peroxide/H₂SO₄ were purchased from Sigma-Aldrich (Darmstadt, Germany). Pure cotton cellulose were obtained from Filmar (Egypt).

### 2. Activation of cotton cellulose serving as substrate

A pure cotton cellulose cloth was activated by immersing them in sodium periodate (NaIO₄) and of sulfuric acid (H₂SO₄) overnight at room temperature to obtained oxidized cellulose. The oxidized cotton cellulose was washed with PBS buffer, subsequently, with cold distilled water, to remove excess of oxidizing agent. Periodate oxidation of cellulose cotton primarily activates hydroxyl groups into aldehyde or carboxyl groups, which will later be coupled with free amine groups of the antibodies or aptamers.

### 3. Immobilization of protease/antibody on the activated cotton

Human respiratory syncytial virus (hRSV) antibody (Ab) and MMP2 protease were coated on the cotton cellulose to later support cleavage of the pathogen. The activated cotton cellulose was incubated into a well-mixed solution of hRSV Ab and PBS and incubated overnight at 4 °C. Accordingly, MMP2 containing coupling buffer was used to couple MMP2 onto the cellulose similar as to the method described for the antibody. Horseredish peroxidase was used for facilitate coupling. The cotton cellulose was washed to remove any unbounded antibody and MMP2 and thereafter 1% BSA was added to block the active sites of the cotton cellulose. A control cotton cellulose cloth was incubated with 1% BSA solution in PBS buffer for 30 min at room temperature.

### 4. Conjugation of Aptamer to blue polymeric microsphere

Blue dyed microsphere with diameter in the range of 300 nm were used. The microsphere contained attached carboxyl groups. hRSV aptamer was washed with distilled water several times before being reacted with a coupling mixture of EDC/NHS overnight. Subsequently, the microspheres were washed with distilled water to remove excess coupling reagent. hRSV aptamer was thus linked to activated blue-dyed microspheres. Blocking of any unbound sites was achieved using 1% BSA solution in PBS for 30 min.

### 5. Attachment of microsphere to the cotton cellulose

The modified cotton cellulose with the attached antibodies from step 3 were washed with PBS followed by immersion the cotton cellulose into a solution of the modified microspheres of step 4, conjugated hRSV aptamer. Reaction was allowed to occur for a a couple of minutes. Subsequently, the cotton was washed with cold PBS to remove any unreacted microsphere. The cotton cellulose was dried and store at room temperature for 24 hrs.

### 6. The assay technique for control

After 24 hrs, the cellulose cotton with the associated microspheres and capture antibodies were exposed to a sample of a patient's salvia, infected with respiratory syncytial virus. A blue color appeared within 3 min, visually displaying the infection.

## Claims

1. Colorimetric detection system for rapid detection of lung diseases comprising antibodies and/or aptamers coupled to a substrate for capturing a lung disease specific antigen, wherein the colorimetric system further comprises dyed microspheres modified with the antibodies and/or aptamers for visually detecting the lung disease specific antigen, **characterized in that** the substrate comprises a protease, preferably a matrix-metalloproteinase.

2. Colorimetric detection system according to claim 1 wherein the dyed microspheres have a mean diameter of less than 6 µm, preferably less than 3 µm, even more preferably less than 1 µm and most preferably less than 500 nm.

3. Colorimetric detection system according to one of the previous claims, wherein the substrate comprises discrete sections, each section being modified with antibodies and/or aptamers different from one of the other section for capturing a lung disease specific antigen different from the one captured in one of the other section.

4. Colorimetric detection system according to one of the previous claims, wherein the antibodies and/or aptamers are influenza virus antibodies and/or aptamers; or a pneumonia or pneumonia-inducing antibodies and/or aptamers.

5. Colorimetric detection system according to one of the previous claims, wherein the substrate is a hydrophilic material and/or oxidized cellulose.

6. A colorimetric face mask comprising a first layer with a colorimetric detection system according to claim 1 to 5.

7. The colorimetric face mask according to claim 6 comprising a second outer layer, preferably comprising a synthetic polymer, most preferably polypropylene.

8. The colorimetric mask according to one of claims 6 to 7 comprising a third inner layer for hiding the result of the visually detected lung disease.

9. The colorimetric mask according to claim 8, wherein the third inner layer is permeable to fluids and preferably comprises a cotton based material.

10. Method for producing a colorimetric detection system according to one of claims 1 to 5, the method comprising the steps of
- Providing a substrate with reactive sites towards antibodies and/or aptamer,
- coupling of antibodies and/or aptamers specific for a lung disease causing antigen to the reactive sites,
- blocking of non-reacted active sites,
- attaching dyed microspheres modified with the antibodies and/or aptamers to the substrate by physical adsorption, preferably by immersing the substrate into a solution comprising the modified dyed microspheres,
**characterized in that** a protease, preferably a matrix metalloproteinase, is coupled to the substrate.

11. Method according to claim 10, wherein two or more different antibodies and/or aptamers each specific for another lung disease causing antigen are coupled to the substrate.

12. Method for producing a mask according to one of claims 6 to 9 comprising the steps:
- Providing a first layer with a colorimetric detection system obtainable by a method according to one of the claims 10 to 11.

13. Method according to claim 12, wherein a second outer layer is provided, preferably an outer layer comprising a synthetic polymer.

14. Use of dyed microsphere modified with antibody and/or aptamer in a colorimetric detection system according to one of claims 1 to 5.

## Patentansprüche

1. Kolorimetrisches Nachweissystem zum schnellen Nachweis von Lungenkrankheiten, umfassend Antikörper und/oder Aptamere, die an ein Substrat zum Einfangen eines lungenkrankheitsspezifischen Antigens gekoppelt sind, wobei das kolorimetrische System ferner gefärbte Mikrokugeln umfasst, die mit den Antikörpern und/oder Aptameren modifiziert sind, um das lungenkrankheitsspezifische Antigen visuell nachzuweisen, **dadurch gekennzeichnet, dass** das Substrat eine Protease, vorzugsweise eine Matrix-Metalloproteinase, umfasst.

2. Kolorimetrisches Nachweissystem nach Anspruch 1, wobei die gefärbten Mikrokugeln einen mittleren Durchmesser von weniger als 6 µm, vorzugsweise weniger als 3 µm, noch bevorzugter weniger als 1 µm und am meisten bevorzugt weniger als 500 nm aufweisen.

3. Kolorimetrisches Nachweissystem nach einem der vorhergehenden Ansprüche, wobei das Substrat diskrete Abschnitte umfasst, wobei jeder Abschnitt mit Antikörpern und/oder Aptameren modifiziert ist, die sich von einem der anderen Abschnitte unterscheiden, um ein lungenkrankheitsspezifisches Antigen einzufangen, das sich von demjenigen unterscheidet, das in einem der anderen Abschnitte eingefangen wurde.

4. Kolorimetrisches Nachweissystem nach einem der vorhergehenden Ansprüche, wobei es sich bei den Antikörpern und/oder Aptameren um Influenzavirus-Antikörper und/oder -Aptamere oder um Lungenentzündung oder Lungenentzündung induzierende Antikörper und/oder Aptamere handelt.

5. Kolorimetrisches Nachweissystem nach einem der vorhergehenden Ansprüche, wobei das Substrat ein hydrophiles Material und/oder oxidierte Cellulose ist.

6. Eine kolorimetrische Gesichtsmaske, umfassend eine erste Schicht mit einem kolorimetrischen Nachweissystem nach einem der Ansprüche 1 bis 5.

7. Die kolorimetrische Maske nach Anspruch 6 umfasst eine zweite Außenschicht, die vorzugsweise ein synthetisches Polymer, am meisten bevorzugt Polypropylen, umfasst.

8. Die kolorimetrische Maske nach einem der Ansprüche 6 bis 7 umfassend eine dritte innere Schicht zum Verbergen des Ergebnisses der visuell erfassten Lungenerkrankung.

9. Die kolorimetrische Maske nach Anspruch 8, wobei die dritte innere Schicht flüssigkeitsdurchlässig ist und vorzugsweise ein Material auf Baumwollbasis umfasst.

10. Verfahren zur Herstellung eines kolorimetrischen Nachweissystems nach einem der Ansprüche 1 bis 5, wobei das Verfahren die folgenden Schritte umfasst
- Bereitstellung eines Substrats mit reaktiven Stellen für Antikörper und/oder Aptamer,
- Kopplung von Antikörpern und/oder Aptameren, die spezifisch für ein eine Lungenerkrankung verursachendes Antigen sind, an die reaktiven Stellen,
- Blockierung von nicht reagierten aktiven Stellen,
- Anbringen von gefärbten Mikrokugeln, die mit den Antikörpern und/oder Aptameren modifiziert sind, an das Substrat durch physikalische Adsorption, vorzugsweise durch Eintauchen des Substrats in eine Lösung, die die modifizierten gefärbten Mikrokugeln enthält,
**dadurch gekennzeichnet, dass** eine Protease, vorzugsweise eine Matrixmetalloproteinase, an das Substrat gekoppelt ist

11. Verfahren nach Anspruch 10, wobei zwei oder mehr verschiedene Antikörper und/oder Aptamere, die jeweils für ein anderes, eine Lungenerkrankung verursachendes Antigen spezifisch sind, an das Substrat gekoppelt werden.

12. Verfahren zur Herstellung einer Maske nach einem der Ansprüche 6 bis 9, umfassend die Schritte:
- Versehen einer ersten Schicht mit einem kolorimetrischen Nachweissystem, erhältlich durch ein Verfahren nach einem der Ansprüche 10 bis 11.

13. Verfahren nach Anspruch 12, wobei eine zweite Außenschicht vorgesehen ist, vorzugsweise eine Außenschicht aus einem synthetischen Polymer.

14. Verwendung von gefärbten, mit Antikörper und/oder Aptamer modifizierten Mikrokugeln in einem kolorimetrischen Nachweissystem nach einem der Ansprüche 1 bis 5.

## Revendications

1. Système de détection colorimétrique pour la détection rapide des maladies pulmonaires comprenant des anticorps et/ou des aptamères couplés à un substrat pour capturer un antigène spécifique d'une maladie pulmonaire, dans lequel le système colorimétrique comprend en outre des microsphères colorées modifiées avec les anticorps et/ou les aptamères pour détecter visuellement l'antigène spécifique d'une maladie pulmonaire, **caractérisé en ce que** le substrat comprend une protéase, de préférence une matrice-métallo-protéinase.

2. Système de détection colorimétrique selon la revendication 1, dans lequel les microsphères colorées ont un diamètre moyen inférieur à 6 µm, de préférence inférieur à 3 µm, de préférence encore inférieur à 1 µm et de préférence inférieur à 500 nm.

3. Système de détection colorimétrique selon l'une des revendications précédentes, dans lequel le substrat comprend des sections distinctes, chaque section étant modifiée par des anticorps et/ou des aptamères différents de l'une des autres sections pour capturer un antigène spécifique d'une maladie pulmonaire différent de celui capturé dans l'une des autres sections.

4. Système de détection colorimétrique selon l'une des revendications précédentes, dans lequel les anticorps et/ou les aptamères sont des anticorps et/ou des aptamères du virus de la grippe, ou des anticorps et/ou des aptamères de la pneumonie ou induisant une pneumonie.

5. Système de détection colorimétrique selon l'une des revendications précédentes, dans lequel le substrat est un matériau hydrophile et/ou de la cellulose oxydée.

6. Un masque facial colorimétrique comprenant une première couche avec un système de détection colorimétrique selon les revendications 1 à 5.

7. Le masque facial colorimétrique selon la revendication 6, comprenant une deuxième couche extérieure, de préférence en polymère synthétique, de préférence en polypropylène.

8. Le masque colorimétrique selon l'une des revendications 6 à 7 comprend une troisième couche interne pour cacher le résultat de la maladie pulmonaire détectée visuellement.

9. Le masque colorimétrique selon la revendication 8, dans lequel la troisième couche interne est perméable aux fluides et comprend de préférence un matériau à base de coton.

10. Un procédé de fabrication d'un système de détection colorimétrique selon l'une des revendications 1 à 5, le procédé comprenant les étapes suivantes
- Fournir un substrat avec des sites réactifs pour les anticorps et/ou les aptamères,
- couplage d'anticorps et/ou d'aptamères spécifiques d'un antigène responsable d'une maladie pulmonaire aux sites réactifs,
- blocage des sites actifs n'ayant pas réagi,
- fixer des microsphères colorées modifiées par les anticorps et/ou les aptamères sur le substrat par adsorption physique, de préférence en immergeant le substrat dans une solution comprenant les microsphères colorées modifiées,
**caractérisé en ce qu'**une protéase, de préférence une métalloprotéinase matricielle, est couplée au substrat.

11. Le procédé selon la revendication 10, dans laquelle deux ou plus anticorps et/ou aptamères différents, chacun spécifique d'un autre antigène responsable d'une maladie pulmonaire, sont couplés au substrat.

12. Un procédé de fabrication d'un masque selon l'une des revendications 6 à 9 comprenant les étapes suivantes :
- Fournir une première couche avec un système de détection colorimétrique obtenu par un procédé selon l'une des revendications 10 à 11.

13. Le procédé selon la revendication 12, dans lequel une deuxième couche extérieure est fournie, de préférence une couche extérieure comprenant un polymère synthétique.

14. Utilisation d'une microsphère colorée modifiée par un anticorps et/ou un aptamère dans un système de détection colorimétrique selon l'une des revendications 1 à 5.
